# EUROPEAN PATENT APPLICATION

(11) **EP 2 005 951 A2**
(43) Date of publication of application: **24.12.2008**
(21) Application number: 07740208.9
(22) Date of filing: 29.03.2007
(51) Int. Cl.: A61K 31/09, A61K 31/122, A61P 25/28, A61P 43/00

(54) **AGENT FOR IMPROVING NERVOUS SYSTEM CELL FUNCTIONS**

(30) Priority: 29.03.2006 JP 2006091490; 16.05.2006 JP 2006136232
(71) Applicant: Kaneka Corporation, Kita-ku Osaka-shi, Osaka 530-8288 (JP)
(72) Inventor: SHINAGAWA, Yoshiyuki, Akashi-shi, Hyogo 674-0051 (JP); KISHIDA, Hideyuki, Kakogawa-shi, Hyogo 675-0039 (JP); KAWABE, Taizo, Himeji-shi, Hyogo 672-8044 (JP); SAKAMOTO, Hirokazu, Kobe-shi, Hyogo 657-0845 (JP); KUBO, Hiroshi, Kobe-shi, Hyogo 655-0046 (JP); HOSOE, Kazunori, Takasago-shi, Hyogo 676-0025 (JP)
(74) Representative: Gillet, Raphaëlle
(86) International application number: PCT/JP2007/056770
(87) International publication number: WO 2007/119578

(57) **Abstract**

The present invention aims to activate neural cells in brain, and further provide a useful composition having an action to protect intracerebral neural cells from external and internal stresses.

An agent for improving a neural cell function containing reduced coenzyme Q as an active ingredient, more specifically, an agent for activating neural cells and an agent for protecting neural cells, is useful, and can be used for the improvement or prophylaxis of central neurological diseases or neuropathy symptoms derived from or associated with a neurodegenerative disease, promotion of cognitive function or improvement of learning and memory disorders.

## Description

### Technical Field

The present invention relates to an agent for improving a neural cell function, more specifically, an agent for activating neural cells and an agent for protecting neural cells, which contain reduced coenzyme Q as an active ingredient or one of active ingredients. Such agents can, for example, promote cognitive function, improve neuropathy symptom or prevent development of symptoms thereof, or improve central neurological diseases and learning and memory disorders.

### Background Art

The brain neural circuit functions by transmission and reception of signals between nerve cells linked by extended protrusions. However, the brain contains not only nerve cells but also glial cells in a number of several to dozen times that of nerve cells. Glial cells support function and survival of nerve cells by surrounding the periphery of nerve cells with glial protrusions, becoming a support for brain tissue construction, supplying nutrition to nerve cells and aiding neurotransmission between nerve cells and the like. The both cells are closely related even during development, born from a common stem cell and control each other's differentiation and growth, and form a dense neural network. When either nerve cell or glial cell is damaged, the normal brain function cannot be maintained.

As neurodegenerative diseases, Parkinson's disease, Alzheimer's disease, dementia and the like are known. Known mechanisms common to the onset of these diseases include low oxygen and low nutrition environment due to ischemia and the like increased concentration of calcium in neural cells (glial cell and nerve cell) caused by excessive glutamic acid and the like, and disorder of cell constituting components due to increased active oxygen species associated therewith (non-patent references 1, 2).

Since the above-mentioned neurodegenerative diseases force psychological pain and economical loss on patients and their families, and markedly impair human Quality of Life (QOL), they are recognized as social problems. At present, however, the response to neurodegenerative diseases and neuropathy symptoms derived therefrom or associated therewith is not sufficient, and there is a demand for an economical and highly effective composition for improving or preventing neurodegenerative diseases or neuropathy symptoms, which shows an enhanced improving effect, a more ensured prophylactic effect, and higher safety at a lower dose. In addition, higher improving effect and higher prophylactic effect on neurodegenerative diseases and neuropathy symptoms derived therefrom or associated therewith are substantially more desirable, and the study of combination agents and combined use for maximization of the effects and minimization of side effects is ongoing.

Coenzyme Q is localized in mitochondria, lysosome, golgi body, microsome, peroxisome, cellular membrane and the like. In mitochondria, it is a substance essential for the functional maintenance of living organisms and known to be involved as a constituent component of an electron transport system in the activation of ATP production, antioxidant action in vivo and stabilization of membrane. In living organisms, coenzyme Q is mostly present in a reduced form, and oxidized coenzyme Q absorbed in the body is intracellularly converted by reductase into a reduced form.

A decrease in the content of coenzyme Q in the body is undesirable for health, since it brings about lowered ATP producibility, lowered heart function, lowered resistance to oxidation stress, and unstabilization of biomembrane. Supply to overcome coenzyme Q shortage is beneficial for promotion of energy production in mitochondria and maintenance of homeostasis by improving antioxidant ability of the body.

It appears that the necessary amount of coenzyme Q is ensured in the normal state, since coenzyme Q is supplemented from diet as well as biosynthesized in the body. However, it is known that the content of coenzyme Q in the body remarkably decreases due to increased age and various stresses the body is exposed to.

Oxidized coenzyme Q10 has long been used as a congestive heart failure drug or a health food, and is a composition with a very few reports of side effects. In a safety test using animals where rat was administered with a high dose of 1.2 g/kg/day for 52 consecutive weeks, no toxicity was observed, and the composition has established high safety (non-patent reference 3).

A report has documented that oxidized coenzyme Q shows a symptom improving effect in a model mouse with Parkinson's disease, which is a representative neurodegenerative disease (non-patent reference 4). It has also been reported that a combined use of oxidized coenzyme Q similarly suppresses an in vitro system in which nerve cell is exposed to hydrogen peroxide to induce cell death (non-patent references 5, 6).

Moreover, for example, a combined use of vitamin E and oxidized coenzyme Q is reported to improve learning and memory disorders in mouse (non-patent references 3, 7).

Furthermore, for example, it is described that a composition of oxidized coenzyme Q, vitamin B6 and iron preparation or a combined use of such compounds is effective for the treatment of dementia (patent reference 1).

In addition, for example, a composition containing oxidized coenzyme Q and lipoic acid is described as a therapeutic agent for dementia (patent reference 2).

Also, for example, a treatment method of memory disorders by a combined use of oxidized coenzyme Q and other agent is described (patent reference 3).

Further, for example, it is described that an aqueous composition of oxidized coenzyme Q is useful for cerebral ischemia and reperfusion injury in rat (patent reference 4).

On the other hand, reduced coenzyme Q10 has been reported to decrease the amount of 8-hydroxy deoxyguanosine, which is an oxidization marker in urine, in normal rat or spontaneously diabetic rat, and further suppress tissue degeneration caused by oxidative stress in the pancreas of diabetic rat (patent reference 5). However, the effectiveness of reduced coenzyme Q10 on the central neurological diseases has not been studied much so far, except a report using coenzyme Q having a content ratio of an oxidized coenzyme Q and reduced coenzyme Q of 2:1. It has been reported that the cell death of astrocytes, one species of glial cell, induced by glutamic acid exposure and low oxygen environment in an in vitro system can be suppressed by a combined use of coenzyme Q (non-patent reference 8).

patent reference 1: JP-B-3064360
patent reference 2: JP 2003-535134
patent reference 3: JP 2003-513039
patent reference 4: JP 2002-541216
patent reference 5: WO2003/032968
non-patent reference 1: Cell Calcium 2003. Vol. 34 385-397
non-patent reference 2: Progress in Neurobiology 2004. Vol. 72 111-127
non-patent reference 3: J. Agric. Food Chem. 1999. Vol. 47 3756-3763
non-patent reference 4: Free Radical Research. 2002. Vol. 36 455-460
non-patent reference 5: Archives of Biochemistry and Biophysics 2004. Vol. 421 54-60
non-patent reference 6: Neurobiology of Disease 2005. Vol. 18 618-627
non-patent reference 7: Free Radical Biology & Medicine, Vol. 38, pp. 729-736 (2005)
non-patent reference 8: Journal of Neuroscience Research 2003. Vol. 72 691-703

### Disclosure of the Invention

### Problems to be Solved by the Invention

It is an object of the present invention to provide an agent for improving a neural cell function.
Specifically, the present invention aims to provide an agent for activating neural cells (particularly an agent for activating intracerebral neural cells), and an agent for protecting neural cells, which protects intracerebral neural cells from external and internal stresses and the like.
More specifically, the present invention aims to provide a cognitive function promoter, an agent for the improvement or prophylaxis of neuropathic symptoms, particularly an agent for the improvement or prophylaxis of neuropathy symptoms derived from or associated with neurodegenerative disease, and an agent for the improvement or prophylaxis of central neurological diseases.

### Means of Solving the Problems

The present inventors have conducted intensive studies in an attempt to achieve the aforementioned objects and found that reduced coenzyme Q has a neural cell function-improving effect, particularly, a neural cell activating action and a neural cell protecting action, activates intracerebral neural cells (glial cell and nerve cell), protects neural cells (glial cell and nerve cell) from external and internal stresses, and moreover, improves central neurological diseases. In addition, they have found that reduced coenzyme Q has an effect of decreasing neuropathy symptoms or preventing development thereof, a cognitive function-promoting effect, and a learning or memory disorder-improving effect.

Accordingly, the present invention provides the following.
[1] An agent for improving a neural cell function, comprising a reduced coenzyme Q represented by the following formula (1):

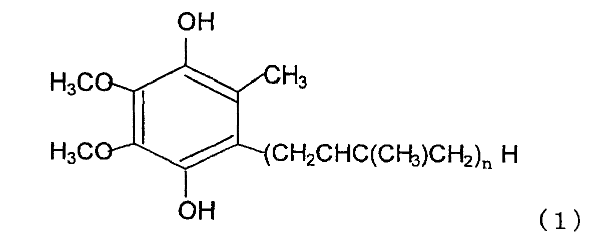

wherein n is an integer of 1 - 12, as an active ingredient.
[2] The agent of [1], which is an agent for activating neural cells.
[3] The agent of [2], which is used for promoting a cognitive function.
[4] The agent of [2], which is used for improving a learning or memory disorder.
[5] The agent of [1], which is an agent for protecting neural cells.
[6] The agent of [2] or [5], wherein the nerve is a central nerve.
[7] The agent of [5], which is used for the improvement or prophylaxis of a neuropathy symptom.
[8] The agent of [7], wherein the neuropathy symptom is at least one symptom selected from the group consisting of ataxia, cognitive functional disorder, disturbance of consciousness, disturbance of sensation, hearing loss, transient blindness, homonymous hemianopsia, aphasia, alternating hemiplegia, contralateral hemiplegia, bilateral quadriplegia, dizziness, tinnitus, hearing disorder, nystagmus, double vision, tremor, numbness in a limb, and coma.
[9] The agent of [5], which is used for the improvement or prophylaxis of a central neurological disease.
[10] The agent of any one of [1] to [9], wherein the reduced coenzyme Q is a reduced coenzyme Q10 (wherein n is 10).
[11] The agent of any one of [1] to [10], wherein the content of reduced coenzyme Q in the agent is 0.001 - 99 wt%.
[12] The agent of any one of [1] to [11], further comprising an oxidized coenzyme Q represented by the following formula (2) :

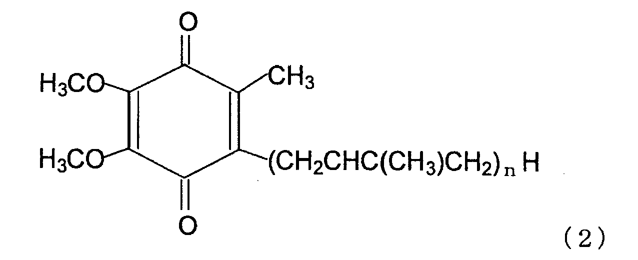

wherein n is an integer of 1 - 12.
[13] The agent of [12], wherein the oxidized coenzyme Q is an oxidized coenzyme Q10 wherein n is 10.
[14] The agent of any one of [1] to [13], additionally comprising at least one compound selected from the group consisting of an acetylcholinesterase inhibitor, a nerve growth factor, dopamine, edaravone, and a tissue plasminogen activator.
[15] The agent of any one of [1] to [13], which targets a patient undergoing a treatment using at least one compound selected from the group consisting of an acetylcholinesterase inhibitor, a nerve growth factor, dopamine, edaravone and a tissue plasminogen activator, a vaccine treatment, or a gene therapy.
[16] A pharmaceutical product comprising the agent of any one of [1] to [15] as an active ingredient.
[17] A quasi-drug comprising the agent of any one of [1] to [15] as an active ingredient.
[18] A food or functional food comprising the agent of any one of [1] to [15].
[19] A nutrition supplement comprising the agent of any one of [1] to [15].
[20] An animal pharmaceutical product comprising the agent of any one of [1] to [15] as an active ingredient.
[21] A pet food, feed or prey comprising the agent of any one of [1] to [15].
[22] A method of improving a neural cell function, comprising a step of administering an effective amount of a reduced coenzyme Q represented by the above-mentioned formula (1) (wherein n is an integer of 1 - 12) to an administration subject.
[23] The method of [22], which is a neural cell activating method.
[24] The method of [23], which is a method of promoting a cognitive function.
[25] The method of [23], which is a method of improving a learning or memory disorder.
[26] The method of [22], which is a method of protecting a neural cell.
[27] The method of [23] or [26], wherein the nerve is the central nerve.
[28] The method of [22], which is a method for the improvement or prophylaxis of a neuropathy symptom.
[29] The method of [28], wherein the neuropathy symptom is at least one symptom selected from the group consisting of ataxia, cognitive functional disorder, disturbance of consciousness, disturbance of sensation, hearing loss, transient blindness, homonymous hemianopsia, aphasia, alternating hemiplegia, contralateral hemiplegia, bilateral quadriplegia, dizziness, tinnitus, hearing disorder, nystagmus, double vision, tremor, numbness in a limb, and coma.
[30] The method of [26], which is a method for the improvement or prophylaxis of a central neurological disease.
[31] The method of any one of [22] to [30], further comprising a step of administering an effective amount of the oxidized coenzyme Q represented by the above-mentioned formula (2) (wherein n is an integer of 1 - 12) to an administration subject.
[32] The method of [22], which is a method for increasing a coenzyme Q concentration in a nerve tissue.
[33] Use of a reduced coenzyme Q represented by the above-mentioned formula (1) (wherein n is an integer of 1 - 12) for the production of the agent of any one of [1] to [11].
[34] Use of a reduced coenzyme Q represented by the above-mentioned formula (1) (wherein n is an integer of 1 - 12) and an oxidized coenzyme Q represented by the above-mentioned formula (2) (wherein n is an integer of 1 - 12), for the production of the agent of [12] or [13].
[35] The agent of any one of [1] to [13], which is used for increasing the concentration of coenzyme Q in a nerve tissue.
[36] A method of selling a composition for improving a neural cell function comprising a reduced coenzyme Q, which comprises use of a packaging container and/or a tool for promoting sales, both indicating that reduced coenzyme Q has a neural cell function improving effect.
[37] The method of [36], wherein the neural cell function improving effect is a neural cell activating action.
[38] The method of [37], wherein the neural cell activating action is a cognitive function promoting action.
[39] The method of [37], wherein the neural cell activating action is a learning or memory disorder-improving effect.
[40] The method of [36], wherein the neural cell function improving effect is a neural cell protecting action.
[41] The method of [40], wherein the neural cell protecting action is a neuropathy symptom improving or preventing action.
[42] The method of [37] or [40], wherein the nerve is that of a central nerve.
[43] A commercial package comprising a composition comprising reduced coenzyme Q as an active ingredient and a written matter associated therewith, the written matter stating that the composition can or should be used for improving a neural cell function.

### Effect of the Invention

Since the agent for improving a neural cell function of the present invention activates neural cells in brain and protects neural cells from external and internal stresses etc., it can improve or prevent central neurological diseases.
Reduced coenzyme Q, which is an active ingredient of the agent for neural cell function of the present invention, has a more superior neural cell or nerve tissue degeneration improving or preventing action, as compared to oxidized coenzyme Q. Therefore, the agent for improving a neural cell function of the present invention can provide a composition that improves neuropathy symptoms or prevents development thereof. In addition, it can provide a safer neural cell function improving agent having particularly high cognitive function promoting effect and learning and memory disorders improving effect, which shows effects rapidly with a smaller amount of use. According to the present invention, moreover, a method of improving a neuropathy symptom or preventing development thereof, a method of promoting a cognitive function and a method of improving learning disorder can be provided. In addition, the agent for improving a neural cell function of the present invention is useful as a pharmaceutical product, a quasi-drug, a food, a functional food, a nutrition supplement, an animal pharmaceutical product, a pet food, a feed or a prey.

### Best Mode for Carrying out the Invention

The present invention is explained in detail in the following.

The agent for improving a neural cell function of the present invention is a composition comprising a reduced coenzyme Q represented by the following formula (1):

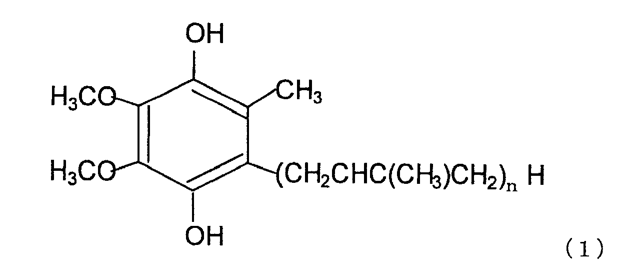

wherein n is an integer of 1 - 12, as an active ingredient. In the present specification, the agent for improving a neural cell function of the present invention is sometimes referred to as a composition containing a reduced coenzyme Q of the present invention or the composition of the present invention.

As mentioned above, coenzyme Q includes oxidized coenzyme Q represented by the following formula (2):

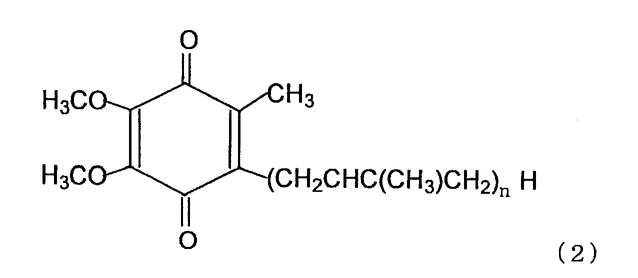

wherein n is an integer of 1 - 12, and reduced coenzyme Q represented by the aforementioned formula (1), and the reduced coenzyme Q is a 2 electron reduced form of oxidized coenzyme Q, which is a benzoquinone derivative widely distributed in the living world.

The composition of the present invention and the methods and sales method using the same contain, as an active ingredient, a reduced coenzyme Q from the above-mentioned coenzymes Q. However, as long as the effect of the present invention is not impaired, it may also contain oxidized coenzyme Q. When a mixture of reduced coenzyme Q and oxidized coenzyme Q is used as the coenzyme Q, the ratio of the reduced coenzyme Q and the oxidized coenzyme Q, the reduced coenzyme Q relative to the whole coenzyme Q is not less than 80% by weight, more preferably not less than 90% by weight, most preferably not less than 95% by weight, in order to maximize the effect of the present invention. The upper limit is not particularly set, and a higher content of the reduced coenzyme Q is more preferable. As a mixture, it is, for example, preferably not more than 99.5% by weight.

The method of obtaining a reduced coenzyme Q or a mixture of a reduced coenzyme Q and an oxidized coenzyme Q, which are the active ingredients of the agent for improving a neural cell function of the present invention, is not subject to any particular limitation. For example, a method including obtaining coenzyme Q containing oxidized coenzyme Q as a main component by a conventionally known method such as synthesis, fermentation, extraction from naturally occurring substances and the like, and concentrating reduced coenzyme Q fraction in an outflow fluid by chromatography as necessary, and the like can be employed. A reduced coenzyme Q can be obtained by adding an ordinary reducing agent such as sodium borohydride or sodium dithionite (sodium hydrosulfite) to the above-described coenzyme Q as required, and reducing the oxidized coenzyme Q in the above-described coenzyme Q by a conventional method to yield a reduced coenzyme Q, which may then be concentrated by chromatography. A reduced coenzyme Q can also be obtained by reacting an existing high-purity oxidized coenzyme Q with the aforementioned reducing agent(s).

Examples of the reduced coenzyme Q as an active ingredient of the agent for improving a neural cell function in the present invention or the oxidized coenzyme Q that can be used as a component of a mixture of the reduced coenzyme Q include those having 1 to 12 side chain repeat units (in the formulas, n) as represented by the formulas (1) and (2) above. In particular, because one having 10 side chain repeat units, i.e., coenzyme Q10, is the primary component of coenzyme Q in the body of humans, as well as in dogs and many other companion animals, coenzyme Q10 can be particularly preferable from the viewpoint of safety and efficacy.

The neural cells in the agent for improving a neural cell function of the present invention mean a cell constituting the central nerve such as brain, spinal cord and the like, or a cell constituting the peripheral nerve such as brain nerve, spinal cord nerve and the like. Specific examples include nerve cell, glial cell (oligodendrocyte, astrocytes, Schwann cell, microglia) and the like. In addition, the neural cell function improvement means an action to activate production of ATP, which is an energy source in the cell, during expression of many cell functions possessed by the neural cells, thereby fulfilling the function more efficiently than in normal conditions, or an action to improve abnormal condition of cell function due to disease, aging and the like to the normal condition of cell function. The agent for improving a neural cell function of the present invention can be used, for example, as an agent for activating neural cells, an agent for protecting neural cells and the like. In the present specification, the agent for activating neural cells and the agent for protecting neural cells are the same concept as a composition for activating intracerebral neural cells and a composition for protecting intracerebral neural cells, each containing reduced coenzyme Q as an active ingredient.

The activation of neural cells for the agent for activating neural cells of the present invention means a state to activate production of ATP, which is an energy source in the cell, during expression of many cell functions possessed by the neural cells, particularly central neuronal cells, specifically intracerebral neural cells (glial cell and nerve cell), thereby fulfilling the function more efficiently than in normal conditions, or improvement of abnormal condition of cell function due to disease, aging and the like to the normal condition of cell function. Therefore, the agent for activating neural cells of the present invention can be used for promoting cognitive functions (as a composition for promoting cognitive function), improving learning and memory disorders and the like.

In addition, the neural cell protection for the agent for protecting neural cells of the present invention means to protect nerve cells, particularly central neuronal cells, specifically intracerebral neural cells (glial cell and nerve cell) from various stresses. Activation of the cell leads to improvement of resistance to various chemical, biological or physical stresses, as a result of which, the cell is protected from various stresses. Therefore, the agent for protecting neural cells of the present invention can be used for the improvement or prophylaxis of neuropathy symptoms (as a composition for the improvement or prophylaxis of a neuropathy symptom), the improvement or prophylaxis of a central neurological disease and the like.

In other words, since the composition containing reduced coenzyme Q10 of the present invention not only protects neural cells (glial cell and nerve cell) from various chemical, biological or physical stresses, but also activates neural cells (glial cell and nerve cell), the composition can prevent disorders due to various stresses from occurring and prevent central neurological diseases. The central neurological disease in the present invention means, from among the neurodegenerative diseases, disorders and diseases caused by lesion in respective parts in the brain or abnormality in the spinal cord. Specific examples of the disease include Alzheimer's disease, Parkinson's disease, Huntington chorea, dementia, cerebral apoplexy and the like. The composition can also be applied to diseases whose symptoms are expected to improve by activating and protecting the nerve cell.

The neuropathy symptom in the present invention is a symptom derived from or associated with neurodegenerative disease. The neurodegenerative disease encompasses any disease that lost normal neurotransmission function and accompanies degeneration of nerve tissue. As the etiology of neurodegenerative disease, for example, hepatic encephalopathy, infection, toxicity, autoimmunity, ischemia and the like are considered. Examples thereof include, but are not limited to, alcoholic poisoning, amyloidosis, HIV, syphilis, virus, autoimmune disease, cancer, porphyria, arachnoiditis, diabetes, toxin, lead, lack of vitamins, chemical substance for paclitaxel chemical therapy, HAART therapy and the like, physical damage such as external injury, pressure, narrowing etc. of nerve tissue, and the like.

Examples of the neurodegenerative disease include, in addition to those mentioned above, spinocerebellar ataxia, dentatorubral-pallidoluysian atrophy, olivo-ponto-cerebellar atrophy, corticobasal degeneration, Diffuse Lewy Body Disease, striatonigral degeneration, choreoathetoid, dystonia, Meige's syndrome, late cortical cerebellar atrophy, familial spastic paraplegia, motor neuropathy, Machado-Joseph Disease, Pick's disease, cerebral apoplexy, cerebral infarction, transient ischemia attack, subarachnoid hemorrhage, nerve dysfunction after brain spinal cord external injury, multiple sclerosis, Guillain-Barre syndrome, acute disseminated encephalomyelitis, acute cerebellitis, transverse myelitis, brain tumor, meningitis, brain abscess, Creutzfeldt-Jakob disease, Korsakoff's syndrome, Down's disease, Parkinson's disease, progressive supranuclear palsy, amyotropic lateral sclerosis retinitis, spinal cord muscular atrophy, glaucoma, epilepsy and Meniere's disease and the like.

The aforementioned nerve tissue means various cells and structure constituent components which constitute the nerve system. It specifically refers to nerve cell, nerve support cell, glial cell, Schwann cell, and vascular cells surrounding the cell group, lymphoid cell, and cerebrospinal fluid, blood and lymph fluid. The nerve tissue may be any nerve tissue in the body, for example, central nerves such as brain, brain stem, spinal cord and the like, peripheral nerve including autonomic nervous system such as sympathetic nerve, parasympathetic nerve and the like.

The neuropathy in the present invention is not particularly limited as long as it is a functional disorder associated with the above-mentioned neurodegenerative disease or various accompanying symptoms, such as peripheral nerve disorder, diabetic neuropathy, autonomic neuropathy and mononeuropathy. Specific examples of the neuropathy symptom include ataxia, cognitive functional disorder, disturbance of consciousness, disturbance of sensation, hearing loss, transient blindness, homonymous hemianopsia, aphasia, alternating hemiplegia, contralateral hemiplegia, bilateral quadriplegia, dizziness, tinnitus, hearing disorder, nystagmus, double vision, tremor, numbness in a limb, coma and the like.

The usefulness for the neuropathy symptom in the present invention means prophylaxis or improvement of neuropathy symptom accompanying a neurodegenerative disease and the like and/or histological and functional recovery accompanying regeneration of nerve cell and other nerve tissues.

The cognitive function in the present invention means stimulation reception sensitivity, processing speed, response speed, concentration, consciousness level or discrimination ability relative to an event selected from the group consisting of auditory stimulation, visual stimulation, olfactory stimulation, taste stimulation and algetic stimulation. Specifically, it refers to a complex process of perceiving external information (sound, word, space, color, people, object, smell, sweet, spicy, sour, bitter, painful, hot, cold etc.), and judging, interpreting and memorizing the external information based on the past experiences and the like, which includes memory, thinking, orientation, comprehension, calculation, learning ability, speech and judgment. The promotion of cognitive function means improvement of sensitivity, processing speed, responsiveness, accuracy, detail in each process of the above-mentioned cognitive function, as well as improvement of accuracy, detail and the like of the short-term and long-term information retention of the information obtained by the cognitive function. Examples thereof include improvement or enhancement of ability relating to learning and memory such as ability to memorize for a short time or a long time, ability to recognize commonness and difference, to acquire new information (to memorize new things in a short time and/or memorize many new things), ability to recognize object, people and space, calculation ability, writing ability, attentiveness, concentration, hearing ability and the like.

The dosage of the agent for improving a neural cell function of the present invention by oral administration (ingestion) is preferably 1 - 1200 mg a day for human (adult) and, from the aspects of usefulness and economy, more preferably 10 - 800 mg and, from the aspect of regular ingestion, particularly preferably 30 - 300 mg, each in the amount of reduced coenzyme Q. When the subject is a child or animal, the above-mentioned preferable dose can be administered after appropriate conversion based on the body weight.

It is known that the above-mentioned dosage to be administered (ingested) varies depending on the dosage form of the preparation. In the case of a highly absorbable preparation, a desired object can also be achieved with a still lower ingestion amount.

The above-mentioned dose per day can be ingested at once or in several portions (e.g., 3 portions).

In the present invention, the above-mentioned dose for one administration (ingestion) can be packed as one unit. For example, when the food is what is called a health food or a pharmaceutical agent, a form wherein the above-mentioned unit dose for one administration (ingestion) is packed and the like can be mentioned. For example, when food is a nutritional drink, a drink containing the above-mentioned dose suspended or dissolved therein is placed in a bottle for a single consumption and the like.

The content of reduced coenzyme Q in the composition of the present invention is not particularly limited as long as its range permits exertion of an objective effect. It is preferably 0.001 - 99 wt%, preferably 0.001 - 60 wt% due to a limitation of preparation making, more preferably 1 - 60 wt% in consideration of a sufficient effect and dose (ingestion amount), and is most preferably 5 - 50 wt% from the aspects of a limitation of preparation making, effect and dose, and practical package of products (combination drug) such as addition of other agents, ingenuity in preparation making and the like, each relative to the total weight of the composition.

With no particular limitation as long as the effect of the present invention is not impaired, other useful compound or extract may be used in combination (compound etc. that can be combined is sometimes to be referred to as a concomitant drug) to increase the useful effect of a composition containing reduced coenzyme Q. For example, a combination of two or more kinds from compound(s) or extract(s) considered to be useful for neurodegenerative diseases and reduced coenzyme Q is considered to show extremely superior usefulness as compared to reduced coenzyme Q alone, since an additive or synergistic effect can be afforded by the combination of useful action mechanisms. For example, such combination is considered to not only decrease neuropathy symptoms but also promote suppression of degeneration of nerve tissues such as protection of nerve cell and/or nerve tissue, and/or regeneration of nerve tissue. The following concomitant drugs may be used alone or in a mixture of two or more kinds thereof.

Examples of those additively or synergistically showing the useful effect as mentioned above together with reduced coenzyme Q include compounds and extracts exhibiting effects of an action to improve the ability of nerve cell, an action to suppress nerve cell and/or nerve tissue disorder and protect nerve tissue, an action to promote regeneration of nerve tissue and the like. While the detail is described later, at least one compound preferably selected from the group consisting of an acetylcholinesterase inhibitor, a nerve growth factor, dopamine, edaravone and a tissue plasminogen activator can be mentioned.

For example, an agent for activating brain function · improving cerebral blood flow and metabolism, an antiepileptic drug, an antiparkinson agent, an antiplatelet agent, an anticoagulant, a thrombolytic agent, an agent effective for the central nervous system (agent for improving dementia etc.), active oxygen eliminator represented by antioxidant and the like can be mentioned. Specific examples thereof include nicergoline, meclofenoxate hydrochloride, ifenprodil tartrate, adenosine triphosphate disodium, ibudilast, dihydroergotoxine mesylate, acetylpheneturide, primidone, ethotoin, sultiame, phenytoin, sodium valproate, carbamazepine, ethosuximide, zonisamide, clobazam, trimethadione, clonazepam, levodopa, bromocriptine, pergolide, talipexole hydrochloride, cabergoline, pramipexole hydrochloride hydrate, selegiline hydrochloride, trihexyphenidyl hydrochloride, biperiden hydrochloride, profenamine hydrochloride, methixene hydrochloride, amantadine hydrochloride, droxidopa, pyrroheptyne, mazaticol hydrochloride, aspirin of non-steroidal anti-inflammatory drug, ibuprofen and diclofenac, ticlopidine hydrochloride, cilostazol, sodium ozagrel of thromboxanesynthase inhibitor, alprostadil, limaprost alfadex warfarin, tiapuride, urokinase, alteplase, a tissue plasminogen activator (tPA), acetylcholine esterase inhibitor such as tacrine, donepezil and the like, mazindol, taltirelin, terguride, calcium hopatenate, piracetam and the like.

Particularly, a combination of reduced coenzyme Q and an acetylcholinesterase inhibitor used for the improvement of dementia such as Alzheimer's disease and the like is particularly preferable, since an additive or synergistic effect can be afforded by the combination of the above-mentioned useful action mechanisms.

In addition, as a compound having a neurotrophic factor activity-enhancing action, a neurodegeneration-suppressive action, a β amyloid toxicity-suppressive action and/or a neurotransmission function-increasing action, for example, a nerve growth factor (NGF) and neurotransmitters such as dopamine or analogs thereof, serotonin, adrenaline, noradrenaline, glutamic acid, acetylcholine, choline, γ-aminobutyric acid and the like can be mentioned. A composition wherein these compounds and reduced coenzyme Q are combined is useful for the improvement or prophylaxis of neuropathy symptoms, or as a composition for promoting cognitive function. Particularly, a combination of NGF and/or dopamine and reduced coenzyme Q is preferable. In addition, a combination of, for example, precursors of neurotransmission or neurotransmitters such as tryptophan, phenylalanine, tyrosine and the like, a calcium antagonist, an NMDA antagonist, an NO synthesis inhibitor and reduced coenzyme Q is also useful as a composition for the improvement or prophylaxis of neuropathy symptoms or a composition for promoting cognitive function.

In addition, as a compound or extract which can be combined with other reduced coenzyme Q, compounds and extracts exhibiting an active oxygen eliminating action, a brain function activating · brain circulation metabolism improving action, a cognitive dysfunction suppressive action, an amyloid lowering action and the like can be mentioned. Specific examples thereof include edaravone, disodium-[(tert-butylamino)methyl]benzene-1,3-disulfonate N-oxide, R-fluribiprofen, probucol, vitamin A, vitamin B and derivatives thereof, vitamin C and derivatives thereof, vitamin E and derivatives thereof, carotenoids, unsaturated fatty acids, polyphenols, glutathione, pyrroloquinolinequinone and pyrroloquinolinequinone derivatives, superoxide dismutase (SOD), glutathione peroxidase, glutathione-S-transferase, glutathione reductase, catalases, ascorbic acid peroxidase, natto kinase, melatonin, curcumin, astaxanthin, huperzine A, N-acetylcysteine and derivatives thereof, taurine, olive oil, phosphatidylserine, St.-John's-wort, terpenes, garlic extract, toki-syakuyakusan, L-carnitine and derivatives thereof, α-lipoic acid and derivatives thereof, gingko leaf extract, iron preparations, zinc, magnesium, selenium, sodium thiosulfate and the like.

Examples of the vitamin B include thiamin, riboflavin, niacin, pantothenic acid, pyridoxine, cyanocobalamine, folic acid, inositol, para-aminobenzoic acid, biotin and the like.

Examples of the carotenoids include licopene, β-carotene, lutein, zeaxanthin and the like.

Examples of the unsaturated fatty acids include DHA, EPA, arachidonic acid and derivatives thereof, γ-linolenic acid and derivatives thereof and the like.

Examples of the polyphenols include flavonol, isoflavone, tannin, catechin, quercetin, anthocyanin, pycnogenol (flavangenol), flavonoids and the like.

Particularly, when improvement of neuropathy symptom derived from or associated with an ischemic neurodegenerative disease such as cerebral infarction and the like is desired, a combine use of reduced coenzyme Q with one or more compounds selected from nizofenone fumarate, trimetazidine hydrochloride, edaravone, disodium-[(tert-butylamino)methyl]benzene-1,3-disulfonate N-oxide and a tissue plasminogen activator is preferable.

While the above-mentioned compound or extract, which is preferably combined with reduced coenzyme Q, can be contained together with reduced coenzyme Q in the composition of the present invention (administration as a single preparation containing the composition of the present invention and a concomitant drug), it can also be administered separately to the target (administration of the composition of the present invention and the concomitant drug as separate preparations). The timing of administration thereof is not limited and they may be administered simultaneously or in a staggered manner to an administration subject. For example, a composition containing reduced coenzyme Q of the present invention can be used for, as a target, a patient undergoing a treatment using the above-mentioned compound or extract (preferably, a treatment using at least one compound selected from the group consisting of an acetylcholinesterase inhibitor, a nerve growth factor, dopamine, edaravone, and a tissue plasminogen activator). Furthermore, a composition containing reduced coenzyme Q of the present invention can also be used for, as a target, a patient undergoing a vaccine treatment or a gene therapy.

The vaccine treatment here is, for example, a treatment aiming at improvement of a neurodegenerative disease via production of an antibody against amyloid beta (Aβ), and means a method including administering a substance such as Aβ and the like as an antigen, administering a DNA vaccine to be expressed in the body and the like.

The gene therapy is, for example, a treatment method that improves neurodegenerative diseases by administering DNA, RNA and the like relating to a substance that causes and/or suppresses a neurodegenerative disease such as neprilysin, dopamine and the like to control expression, production, secretion and metabolism of the causative or related substance of neurodegenerative diseases. In addition, the gene therapy also includes, for example, a cell therapy that transfuses a cell etc. that control expression, production, secretion and metabolism of a causative or related substance of neurodegenerative diseases.

The composition of the present invention may be further added and mixed with, besides reduced coenzyme Q or a mixture of reduced coenzyme Q and oxidized coenzyme Q, pharmaceutically and food hygienically acceptable other materials appropriately by a conventional method. Examples of such materials include, but are not limited to, an excipient, a disintegrant, a lubricant, a binder, a coating agent, a colorant, an anticoagulant, an absorption promoter, a solubilizer, a stabilizer, a health food material, a nutritional supplementary food material, a vitamin, a flavor, a sweetening agent, an antiseptic, a preservative, an antioxidant and the like. In addition, when the composition is used as a countermeasure for various diseases, it can also be combined with a known pharmaceutical agent for the disease.

The above-mentioned excipient is not particularly limited and, for example, sucrose, lactose, glucose, cornstarch, mannitol, crystalline cellulose, calcium phosphate, calcium sulfate and the like can be mentioned.

The above-mentioned disintegrant is not particularly limited and, for example, starch, agar, calcium citrate, calcium carbonate, sodium hydrogen carbonate, dextrin, crystalline cellulose, carboxymethylcellulose, tragacanth and the like can be mentioned.

The above-mentioned lubricant is not particularly limited and magnesium stearate, sodium stearate, stearic acid, calcium stearate, magnesium oleate, oleic acid, potassium oleate, caprylic acid, sodium stearyl fumarate , magnesium palmitate and a blend of such lubricant and the like can be mentioned.

The above-mentioned binder is not particularly limited and starch and derivatives thereof (pregelatinized starch, dextrin etc.), cellulose and derivatives thereof (ethylcellulose, sodium carboxymethylcellulose, hydroxypropylmethylcellulose etc.), gum arabic, tragacanth, gelatin, saccharides (glucose, sucrose etc.), ethanol, polyvinyl alcohol and the like can be mentioned.

The above-mentioned coating agent is not particularly limited and cellulose derivatives (hydroxypropylcellulose, cellulose acetate phthalate, hydroxypropylmethylcellulosephthalate etc.), shellac, polyvinylpyrrolidone, polyvinyl pyridines (poly-2-vinylpyridine, poly-2-vinyl-5-ethylpyridine etc.), polyvinyl acetyldiethylaminoacetate, polyvinyl alcohol phthalate, methacrylate·methacrylic acid copolymer and the like can be mentioned.

The above-mentioned colorant is not particularly limited and those admitted for addition to pharmaceutical products, foods and the like can be used. Examples thereof include food blue No. 1, food yellow No. 4, food green No. 3, food red No. 5, lake pigment, titanium dioxide, red cabbage pigment, monascus color, purple sweet potato color, gardenia pigment, cochineal extract and the like.

The above-mentioned anticoagulant is not particularly limited and those admitted for addition to pharmaceutical products or foods can be used. Examples thereof include stearic acid, talc, light anhydrous silicic acid, hydrated silicon dioxide etc. and the like.

The above-mentioned absorption promoter is not particularly limited and, for example, higher alcohols, higher fatty acids, surfactants such as glycerolfatty acid ester and the like can be mentioned.

The above-mentioned solubilizing agent is not particularly limited and, for example, adipic acid, L-arginine, sodium benzoate, benzyl benzoate, esterification corn oil, ethanol, magnesium chloride, hydrochloric acid, olive oil, carmellose sodium, dried sodium carbonate, diluted hydrochloric acid, citric acid, sodium citrate, glycine, glycerol, glycerol fatty acid ester, geraniol, sesame oil, cellulose acetate phthalate, sodium salicylate, magnesium oxide, α-cyclodextrin, β-cyclodextrin, γ-cyclodextrin, dibutylhydroxytoluene, tartaric acid, sucrose fatty acid ester, sodium hydroxide, sorbitan sesquioleate, sorbitan fatty acid ester, D-sorbitol, D-sorbitol solution, soybean oil, soybean lecithin, sodium hydrogen carbonate, sodium carbonate, medium-chain triglyceride, triacetine, sorbitan trioleate, nicotinic-acid amide, lactic acid, concentrated glycerin, white copper, hydroxypropylmethylcellulose, castor oil, glacial acetic acid, glucose, propylene glycol, propylene glycol fatty acid ester, povidone, polyoxyethylene hydrogenated castor oil, polyoxyethylene(160)polyoxypropylene(30)glycol, polysorbate, polyvinyl alcohol, macrogol, D-mannitol, myristic acid isopropyl, dehydrated ethanol, citric anhydride, monooleic acid sorbitan, lauryl macrogol, lidocain, phosphoric acid, sodium hydrogen phosphate, potassium dihydrogen phosphate and the like can be mentioned.

The above-mentioned stabilizer is not particularly limited and, for example, benzoic acid, sodium benzoate, ethyl parahydroxybenzoate and the like can be mentioned.

The above-mentioned health food material is not particularly limited and, for example, kanpo medicines (e.g., ireito, unkeito, unsei'in, ogi-kentyuto, oren-gedokuto, orento, kakkonto, kami-kihito, kami-syoyosan, kanbaku-daisoto, kikyoto, kihito, kyumi-binroto, keigai-rengyoto, keisi-ka-syakuyaku-daioto, keihi-ka-syakuyakuto, keihi-ka-ryukotu-boreito, keisito, keisi-ninzinto, keisi-bukuryogan, keihito, kososan, gokoto, gosyakusan, gosha-jinkigan, gorinsan, saikanto, saiko-ka-ryukotu-boreito, saiko-keisi-kankyoto, saiko-keisito, saiko-seikanto, saibokuto, saireito, sansoninto, ziin-kokato, sigyakusan, sikunsito, simotuto, sya-kanzoto, syakuyakukanzoto, zyuzen-taihoto, zyumi-haidokuto, syoken-tyuto, syosaikoto, syoseiryuto, syohusan, sin'i-seihaito, sinpito, sinbuto, seizyo-bohuto, seisyo-ekkito, seisin-rensiin, seihaito, sokei-kakketuto, daio-kanzoto, daio-botanpito, daikentyuto, daisaikoto, daisaikoto-kyo-daio, daijyokito, daibohuto, jidaboku-ippo, tyoi-zyokito, tyotosan, tyoyoto, tyoreito, tyoreito-go-simotuto, tudosan, tokaku-zyokito, toki-insi, toki-kentyuto, toki-syakuyakusan, tokito, nitinto, nyosinsan, ninzinto, ninzin-yoeito, hainosankyuto, bakumondoto, hatimiziogan, hange-kobokuto, hange-syasinto, byakko-ka-ninzinto, bukuryoin, bukuryoin-go-hange-kobokuto, heiisan, boi-ogito, bohu-tusyosan, hotyu-ekkito, maoto, mao-busi-saisinto, makyo-kansekito, masiningan, mokuboito, yokukansan, yokukansan-katinpi-hange, rikkunsito, rikkosan, ryutan-syakanto, ryokankyomi-singeninto, rokumi-ziogan etc.), tea leaves (e.g., green tea, unmilled rice tea, powdered tea, green tea of middle grade, toasted tea, roasted tea, jasmine tea, oolong tea, hongcha, heicha, huacha, jincha, baicha etc.), herbs (e.g., Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, European linden, scented geranium, St. John's wort, soapwort, Solomon's-seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemon grass, lemon verbena, lemon balm, rose, rosemary, rocket, wild strawberry, wild pansy, forget-me-not etc.), propolis, ginkgo leaf, aojiru (green-leaved-vegetable juice) and extract thereof and the like can be mentioned.

The nutritional supplementary food material is not particularly limited and amino acids, metal ions, proteins, saccharides, fatty acids, yeast extract, vegetable extract, fish meat extract, fruit, fruit extract, N-acetylglucosamine, S-adenosylmethionine, tetrahydrobiopterin and the like can be mentioned.

The vitamin is not particularly limited and, for example, vitamin A, vitamin B, vitamin C, vitamin D, vitamin E, vitamin K, derivatives thereof and the like can be mentioned.

Examples of the flavor include single-ingredient flavors such as menthol, carvone, anethole, cineole, methyl salicylate, cinnamic aldehyde, eugenol, 3,1-menthoxypropane-1,2-diol, thymol, linalol, linalyl acetate, limonene, menthone, menthyl acetate, N-subsituted-para-menthane-3-carboxamide, pinene, octylaldehyde, citral, pulegone, carvyl acetate, anise aldehyde, ethyl acetate, ethyl butyrate, arylcyclohexane propionate, methyl anthranylate, ethylmethylethinyl glycidate, vanillin, undecalactone, hexanal, ethyl alcohol, propyl alcohol, butanol, isoamyl alcohol, hexenol, dimethyl sulfide, cyclotene, furfural, trimethylpyrazone, ethyl lactate, and ethyl thioacetate; as well as natural flavors such as peppermint oil, spearmint oil, anise oil, eucalyptus oil, wintergreen oil, cassia oil, clove oil, thyme oil, sage oil, lemon oil, orange oil, mentha oil, cardamom oil, coriander oil, mandarin oil, lime oil, lavender oil, rosemary oil, laurel oil, chamomile oil, caraway oil, marjoram oil, bay oil, lemon-grass oil, origanum oil, pine needle oil, neroli oil, rose oil, jasmine oil, iris concrete, absolute peppermint, absolute rose, and orange flower; blended flavors such as strawberry flavor, apple flavor, banana flavor, pineapple flavor, grape flavor, mango flavor, butter flavor, milk flavor, fruit mix flavor, and tropical fruit flavor, etc. and the like.

Examples of the sweetener include saccharin sodium, aspartame, stevioside, stevia extract, para-methoxycinnamic aldehyde, neohesperidyl dihydrochalcone, perilla rutin and the like.

Examples of the antiseptic include aminoethylsulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white soft sugar, honey, isobutyl p-hydroxybenzoate, isopropyl p-hydroxybenzoate, ethyl p-hydroxybenzoate, butyl p-hydroxybenzoate, propyl p-hydroxybenzoate, methyl p-hydroxybenzoate, l-menthol, eucalyptus oil and the like.

Examples of the preservative include benzoic acid, sodium benzoate, ethanol, sodium edetate, dry sodium sulfite, citric acid, glycerol, salicylic acid, sodium salicylate, dibutylhydroxytoluene, D-sorbitol, sorbic acid, potassium sorbate, sodium dehydroacetate, isobutyl para-oxybenzoate, isopropyl para-oxybenzoate, ethyl para-oxybenzoate, butyl para-oxybenzoate, propyl para-oxybenzoate, methyl para-oxybenzoate, propylene glycol, phosphoric acid and the like.

Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α lipoic acid and derivatives thereof, pycnogenol, flavangenol, superoxide dismutase (SOD), glutathione peroxidase, glutathione-S-transferase, glutathione reductase, catalases, ascorbic acid peroxidase, and mixtures thereof and the like. In particular, antioxidants are used as additives particularly preferably from the viewpoint of increasing the oxidative stability of the reduced coenzyme Q, and hence obtaining stabler and higher effectiveness.

The content, dosage form, preservation method and preservation form of reduced coenzyme Q when producing a composition containing reduced coenzyme Q of the present invention can be appropriately determined according to the use thereof. The composition of the present invention can be used for treatment and medicinal purposes such as for decreasing neuropathy symptoms derived from or associated with neurodegenerative diseases or preventing the development thereof, for promoting cognitive function, and the like, as well as for daily maintenance, promotion and the like of health.

The use, dosage, delivery technology, delivery route (administration method), carrier for delivery, formulation and the like of the composition of the present invention are not particularly limited, and determined as appropriate within the range not impairing the effect of the invention, in consideration of the effectiveness, convenience, economy, versatility, safety and the like. Depending on the compound or extract to be used in combination or prepared into a combination drug, the use, dosage, delivery technology, delivery route (administration method), carrier for delivery, formulation and the like can be changed as appropriate within the range not impairing the effect of the invention, in consideration of the effectiveness, convenience, economy, versatility, safety and the like. Furthermore, depending on the patients or consumers who are administered with or ingest the composition, the use, dosage, delivery technology, delivery route (administration method), carrier for delivery, formulation and the like can be changed as appropriate within the range not impairing the effect of the invention, in consideration of the effectiveness, convenience, economy, versatility, safety and the like.

Examples of the administration method include oral, intravenous, dermal, subcutaneous, intramuscular, sublingually, pulmonary, transmucosal, transnasal and the like. Depending on the administration route, moreover, simultaneous or continuous administration is possible. When the object is improvement of acute neuropathy symptom, rapid expression of effect, expression of strong effect or expression of usefulness in a short time, bolus administration such as intravenous administration and the like is preferable. In addition, when the object is reduction of neuropathy symptoms or prevention of development thereof over medium - long term, and promotion of cognitive function, oral administration (ingestion) is preferable. As the subject of administration or ingestion, for example, mammals including human, primates, horse, birds, bovine, pig, dog, cat and mouse can be mentioned, with preference given to human.

The composition of the present invention can be widely used for pharmaceutical products, quasi-drugs, foods and functional foods, as well as nutrition supplements, health foods, cosmetics, pharmaceutical products for animals, supplements for animals, pet foods, feeds for animals, preys for fish etc. and the like.

The composition containing reduced coenzyme Q of the present invention can be directly used as a pharmaceutical product or a quasi-drug, or as a mixture with other pharmaceutically acceptable components. Examples of the preparation form for use as a pharmaceutical product or a quasi-drug include injection, drip infusion, syrup, external liquid, jelly, ointment, endermic liniment, inhalant, aerosol, spray, suppository, rectal capsule, impregnating agent, pessary for vaginal administration and the like, as well as various preparations for oral administration. Examples of the application of injection and drip infusion include intravenous, intramuscular, subcutaneous, intraorgan, intranasal, intradermal, instillation, intracerebral, intraperitoneal, lesion and the like. Examples of the application of impregnating agent include rectal, vaginal and the like. Examples of the various preparations for oral administration include tablet, pill, capsule, powder, fine granule, granule and the like. Of the above-mentioned preparations, preferred are oral agent, inhalant, impregnating agent, drip infusion and injection, and more preferred are oral agent mainly for internal use, drip infusion and injection.

When the composition of the present invention is used as a pharmaceutical product or a quasi-drug, or ingested as food and drink, the object is, for example, improvement of neuropathy symptoms or prevention of the development of neuropathy. Alternatively, it may be, for example, improvement or prevention of central neurological diseases, promotion of cognitive ability, and improvement of learning and memory disorders.

Particularly, the composition of the present invention has an action to prevent a decrease of, improve or enhance normal response of cognitive ability, an action to prevent a decrease of, improve or enhance normal response of consciousness level, and an action to prevent a decrease of, improve or enhance normal response of distinguishing ability of events (auditory stimulation, visual stimulation, olfactory stimulation, taste stimulation, algetic stimulation). Therefore, the composition is effective as a food and drink, a pharmaceutical product, a quasi-drug and the like. In addition, an action to prevent a decrease of, improve or enhance normal response of cognitive ability is effective for a food and drink, a pharmaceutical product and a quasi-drug aiming at an action to prevent a decrease of, improve or enhance normal response such as attention, memory, perception, language, calculation and the like, and further, for a food and drink, a health food, a functional food, a food for specified health uses, a food for infants, a food for elder and the like aiming at maintenance or improvement of concentration, maintenance of attentiveness, clearing up one's head, fully wakeful head, rejuvenation and the like.

In addition, the composition containing reduced coenzyme Q of the present invention is also directly used as a food or a functional food or as a mixture with other foods and acceptable components. The food and the functional food are not particularly limited. From the aspects of daily, effective and long-term ingestion of reduced coenzyme Q, however, the composition is preferably used for fluid foods such as beverages (e.g., refreshing beverages, milk beverages, vegetable/fruit juice beverages, alcoholic beverages, tea), health drink and soup; milky or pasty foods such as curry; semi-solid foods such as jelly and "gumi" candies; spontaneously liquid diets, semi-digested nutritional foods and component nutrient foods; and the like.

When the composition containing reduced coenzyme Q of the present invention is used for the above-mentioned pharmaceutical products, quasi-drugs, foods, functional foods and the like, the form of the composition can be appropriately selected from an aqueous solution state, a powder state, a liquid state and the like according to the form and intended use thereof.

The present invention provides a sales method of a composition containing reduced coenzyme Q for improving a neural cell function, comprising using a packaging container and/or a tool for promoting sales, which indicates that reduced coenzyme Q has a neural cell function improving effect. To be precise, the food or functional food containing the composition of the present invention, such as food and drink, health food, food for specified health uses, food with nutrient function claims, food for infants, food for elders and the like, can be sold using a packaging container and/or a tool for promoting the sales thereof (e.g., brochure etc.), which describe or indicate that the food or functional food containing the composition of the present invention has a neural cell function improving effect (e.g., neural cell activating action, neural cell protecting action), an action to improve neuropathy symptom or prevent the development thereof, an action to improve or enhance cognitive ability and an action to improve central neurological disease. In this case, the food or functional food containing the composition of the present invention may be sold using a packaging container and/or a tool for promoting the sales thereof (e.g., brochure etc.), which describe or indicate, as another expression of the above-mentioned actions, that it has an action to prevent a decrease of, improve or enhance normal response of consciousness level; an action to prevent a decrease of, improve or enhance normal response of discrimination ability of events (auditory stimulation, visual stimulation, olfactory stimulation, taste stimulation, algetic stimulation); an action to prevent a decrease of, improve or enhance normal function such as attention, memory, perception, language, calculation and the like; an action of maintenance or improvement of concentration, maintenance or improvement of attentiveness, clearing up one's head, fully wakeful head, rejuvenation and the like.

Specific examples of the method of describing or indicating on the above-mentioned packaging container and/or a tool for promoting sales (including electronic medium such as internet etc.) include clearly describing the prophylactic or improving effect for neuropathy symptoms or promoting effect of cognitive function achieved by the use of the present composition, or making target patients or consumers conscious of such effects or implying such effects to them by using photograph of brain or illustration giving an image of the brain; using photograph or illustration of smiles of seniles, children and women, happy life of a family-like group and the like; presenting an opinion and/or photograph or illustration of socially qualified person or authority (doctor, pharmacist, dietician etc.) having high social confidence or considered by patients or consumers to have high social confidence; and the like.

Specific examples of the expression relating to the improvement of neuropathy symptoms or prevention of the development thereof include expressions such as easy to move, light, feeling young again, without feeling age, can concentrate and having more incentive. Specific examples of the expression relating to the promotion of cognitive ability include expressions such as improvement of memory loss, prophylaxis and improvement of dementia, improvement of a symptom of disorientation, and feeling refreshed. In addition, the ability to remember, for example, name, place and words tends to decrease with aging, and therefore, expressions such as suppressing such decrease, brain-friendly, forever youthfully, being quite well, decreasing memory loss, prevention of dementia, keeping sharp, refreshing brain, enhancing concentration, improving memory, activating brain function, brain benefit, important action on intracerebral (nerve etc.) cells, preventing aging of brain, capable of enhancing energy production in the brain and the like can be mentioned.

Specific examples of the expressions common to the improvement of neuropathy or prevention of the development of neuropathy, and promotion of cognitive ability include expressions such as smoothly speaking and free of stuttering.

While the involvement mechanism that affords usefulness by ingestion of reduced coenzyme Q is not particularly limited, it is considered, for example, that ingestion (administration) of a composition containing reduced coenzyme Q eliminates free radical and suppresses lipoperoxidation, whereby oxidative disorder of brain cells (vascular endothelial cell, nerve cell) is suppressed. For such useful effects of reduced coenzyme Q to be expressed, it is considered that total coenzyme Q concentration in the brain tissue must have increased, or the proportion of reduced coenzyme Q to oxidized coenzyme Q must have increased, or both. It is assumed that, as a result of the direct and/or indirect effect by increased total coenzyme Q concentration in the nerve tissue and/or increased proportion of reduced coenzyme Q to oxidized coenzyme Q, the cognitive function promoting action has expressed. In other words, it is considered that the composition of the present invention is also useful as a composition for increasing coenzyme Q concentration in the nerve tissue, and the administration of the composition of the present invention can increase coenzyme Q concentration in the nerve tissue of the subject.

Moreover, as other mechanism that affords usefulness by such ingestion of reduced coenzyme Q, it is considered that the function and structure of the nerve tissue necessary for the promotion and maintenance of cognitive function are protected or promoted via direct and indirect action of reduced coenzyme Q in the body, although not necessarily requiring direct correlation between increased total coenzyme Q concentration in the nerve tissue and/or increased proportion of reduced coenzyme Q to oxidized coenzyme Q. Particularly, administration or ingestion of reduced coenzyme Q is considered to suppress degeneration of the nerve tissue and decrease in the nerve tissue function in the olfactory bulb in the brain and spinal cord, cerebral cortex, hippocampus, corpus striatum, thalamus, diencephalon, mesencephalon, cerebellum, pons, medulla oblongata, spinal cord and retinal region, which are associated with brain edema, cerebral infarction, neural symptoms, tardive nerve cell death and the like.

On the other hand, there is a possibility that the nerve tissue function is promoted, due to which it is considered that the amount and presence of enzymes relating to neurotransmitters (e.g., acetylcholinetransferase and the like) and neurotransmitters themselves in the brain have been changed. The test results in the below-mentioned Examples have confirmed by a passive avoidance test that reduced coenzyme Q suppresses or improves the progress of learning and memory disorders, in SAMP8 mouse showing learning and memory disorders accompanied by degeneration of the central nerve. Thus, reduced coenzyme Q promoted cognitive function, i.e., normal response reaction enabling to distinguish and recognize a dark compartment and a light compartment, and recognize that the dark compartment is dangerous. It is considered that, for the process of recognizing that the dark compartment is dangerous, normal response of the ability to discriminate events (auditory stimulation, visual stimulation, olfactory stimulation, taste stimulation, algetic stimulation) has been promoted. In addition, the maintenance of the information that the dark compartment is dangerous is considered to have been promoted by the usefulness of the ingestion of reduced coenzyme Q established in the below-mentioned Examples. For the maintenance of information, moreover, it is considered that normal response at the consciousness level to integrate and judge events (auditory stimulation, visual stimulation, olfactory stimulation, taste stimulation, algetic stimulation) has been promoted.

It is also considered that the usefulness of the ingestion of reduced coenzyme Q established in the present Examples promoted an appropriate judgment (normal response) to recall (remember) the maintained event memory, and not enter the dark compartment. Furthermore, for example, reduced coenzyme Q acts during the cerebral infarction acute stage and shows a brain protective action by suppressing the expression and progression (aggravation) of ischemic cerebrovascular disorders such as brain edema, cerebral infarction, neural symptoms, tardive nerve cell death and the like. It is considered that a brain nerve protecting action, a suppressive action on decreased blood flow in regions near the infract zone, a brain protective action (brain edema and cerebral infarction suppressive action, neural symptoms reducing action) and a tardive nerve cell death suppressive action are obtained. Moreover, direct and indirect elimination of free radicals and lipoperoxidation suppressive action, tissue disorder suppressive actions such as vascular endothelial cell disorder suppressive action and the like of reduced coenzyme Q as mentioned above are considered to be further involved.

From the above, the usefulness of oral administration or ingestion of a composition containing reduced coenzyme Q is evident, and the high safety of reduced coenzyme Q itself is ideal for pharmaceutical products, quasi-drugs, foods and functional foods when improvement of neuropathy symptoms, prophylaxis of the development of neuropathy, and further, promotion of cognitive function are desired over a medium to long period.

The composition of the present invention can be directly administered to animals or human, or as a pharmaceutical composition containing a conventional carrier for pharmaceutical preparations.

Useful dosage forms for pharmaceuticals include oral preparations (liquid preparations such as extracts, elixirs, syrups, tinctures, and lemonades; solid preparations such as capsules, granules, pills, powders and tablets), injections, nasal drops, eye drops, suppositories, sprays, and dosage forms for transdermal administration, such as ointments and patches.

Furthermore, for edible uses, the composition containing reduced coenzyme Q of the present invention may be directly taken, or a food or drink containing the same may be taken.

As a nutrition supplement, the composition containing reduced coenzyme Q of the present invention may be directly taken, or a food or drink containing the same may be taken. Furthermore, the composition can be used as a health food or a nutritional supplementary food in the same manner.

Useful dosage forms for animal drugs include oral preparations (liquid preparations such as extracts, elixirs, syrups, tinctures, and lemonades; solid preparations such as capsules, granules, pills, powders, and tablets), injections, nasal drops, eye drops, suppositories, and preparations for transdermal administration, such as ointments and patches.

As a pet food, feed or prey, the composition containing reduced coenzyme Q of the present invention may be directly fed, or a food or drink containing the same may be fed. Furthermore, the composition can be used as a health food or a nutritional supplementary food for animals in the same manner.

The present invention provides a method of improving neural cell function, a method of activating neural cells, a method of promoting a cognitive function, a method of protecting neural cells, a method for the improvement or prophylaxis of neuropathy symptoms, a method for the improvement or prophylaxis of central neurological diseases or a method of increasing the concentration of coenzyme Q in a nerve tissue, each of which comprises a step of administering an effective amount of reduced coenzyme Q and/or oxidized coenzyme Q to an administration subject.

When reduced coenzyme Q and oxidized coenzyme Q are to be administered (ingested), they may be administered as a single preparation obtained by simultaneously formulating reduced coenzyme Q and oxidized coenzyme Q, or two kinds of preparations obtained by separately formulating reduced coenzyme Q and oxidized coenzyme Q may be simultaneously administered in combination or in a staggered manner, as long as they can be combined at the time of administration.

A commercial package comprising a composition comprising reduced coenzyme Q as an active ingredient and a written matter (e.g., manual for performing the above-mentioned method using a kit for performing the above-mentioned method) associated therewith, the written matter stating that the composition can or should be used for improving a neural cell function is also included.

### Examples

The present invention is hereinafter described in more detail by means of the following examples, which, however, are not to be construed as limiting the scope of the invention.

### (Production method 1) Production of Reduced Coenzyme Q10

100 g of oxidized coenzyme Q10 (purity 99.4%) and 60 g of L-ascorbic acid were added to 1000 g of ethanol, and a reducing reaction was performed with stirring at 78°C. After 30 hours, the reaction mixture was cooled to 50°C, and while maintaining the temperature, 330 g of ethanol and 70 g of water were added. While stirring, this ethanol solution (containing 100 g of reduced coenzyme Q10) was cooled to 2°C at a cooling rate of 10°C/hour to yield a white slurry. This slurry was filtered under reduced pressure; the resulting wet crystal was sequentially washed with cold ethanol, cold water, and cold ethanol in this order (the cold solvents for the washing were used at 2°C), and the wet crystal was dried under reduced pressure (20 to 40°C, 1 to 30 mmHg) to yield 97 g of a white dry crystal. All operations but drying under reduced pressure were performed in a nitrogen atmosphere.

### (Preparation Example 1) Preparation of liposome containing reduced coenzyme Q10

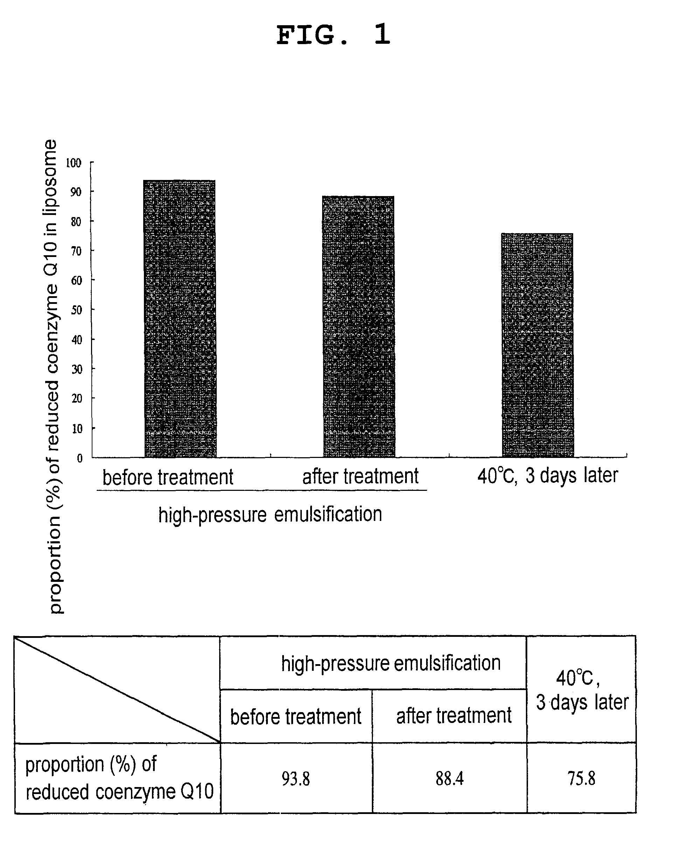

As a lipid component of a liposome, 204 mg of soybean-derived lecithin (containing phosphatidylcholine, NIPPON FINE CHEMICAL CO., LTD.) and 10.5 mg of cholesterol (Wako Pure Chemical Industries, Ltd.) were added to a 100 ml eggplant flask. Further, 20 ml of chloroform (Wako Pure Chemical Industries, Ltd.) was added to dissolve the lipid. Further, 50 mg of reduced coenzyme Q10 (containing 3% of oxidized coenzyme Q10) was added, and the mixture was dissolved by heating at 60°C. After complete dissolution, chloroform was evaporated by a rotary evaporator to concentrate the dissolved components. After confirmation of lipid film formation, eggplant flask was placed in a desiccator, and chloroform was evaporated by suction using a vacuum pump for 2 hr. Chloroform was completely evaporated, 10 ml of distilled water was added, and the lipid film was dispersed by an ultrasonication apparatus to give an aqueous liposome solution. The uniformly dispersed aqueous liposome solution was processed into fine particles by a high-pressure emulsifying machine, and the emulsifying operation was repeated until the median size of the liposome particle size reached not more than 100 nm. The aqueous liposome solution used as a control was free of coenzyme Q10. The prepared aqueous liposome solution was preserved at 4°C. The ratio of reduced coenzyme Q10 and oxidized coenzyme Q10 present in the liposome the prepared coenzyme Q10 was measured by high performance liquid chromatography. Furthermore, the proportion of reduced coenzyme Q10 after incubation at 40°C for 3 days was measured, and the oxidization stability of the liposome containing reduced coenzyme Q10 was examined. The results are shown in Fig. 1. The proportion of reduced coenzyme Q10 to the total of coenzyme Q before high-pressure emulsion treatment was 93.8%. It was 88.4% after the high-pressure emulsion treatment, and 75.8% after the incubation at 40°C for 3 days.

### (Preparation Example 2) Preparation of liposome containing oxidized coenzyme Q10

In the same manner as in Preparation Example 1 except that oxidized coenzyme Q10 (100%) was used instead of reduced coenzyme Q10, liposome containing oxidized coenzyme Q10 was prepared.

### (Example 1) Cell activating effect of reduced coenzyme Q10

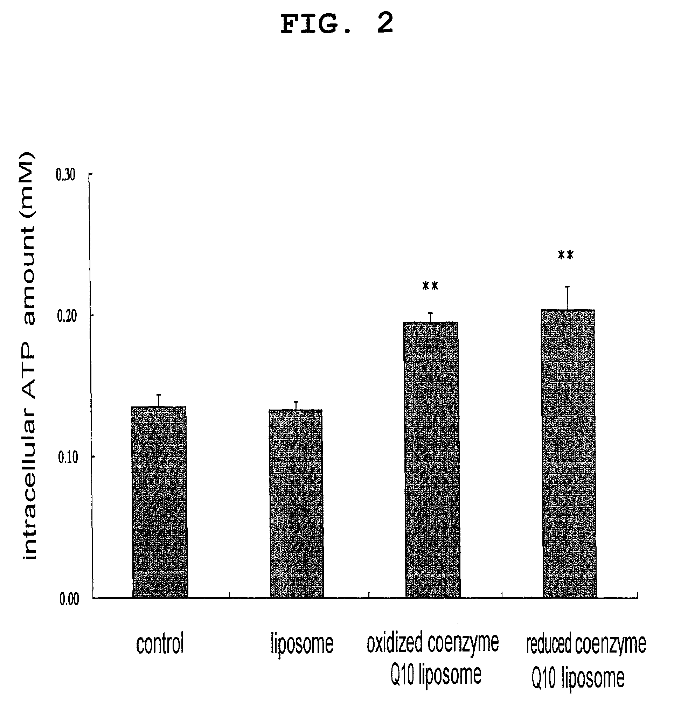

Human glioma-derived cells (hereinafter to be abbreviated as GI-1, supplied by RIKEN) were passage cultured in Dulbecco's Modified Eagle Medium containing 200 units of penicillin, 0.1 µg/ml streptomycin, and 10% fetal bovine serum under 37°C, 5% CO₂ conditions. 5×10⁶ cells were inoculated to a 10 cm dish, cultured to confluent. When the cells became confluent, the medium was substituted with one containing 0.5% fetal bovine serum, and reduced coenzyme Q10 liposome or oxidized coenzyme Q10 liposome was added to adjust the coenzyme Q10 concentration to 50 µg/ml. As the control, cells free of addition and cells only added with liposome free of coenzyme Q10 were used. Using a shaker, the cells were cultured for 2 days while stirring the medium. The medium was removed, and the cells were washed 3 times with PBS, detached with trypsin and recovered by centrifugation. The recovered cells were re-suspended in Dulbecco's Modified Eagle Medium (containing 10% fetal bovine serum), plated in a 96 well microplate at 1×104 cells/well, and cultured under the conditions of 37°C, 5% CO₂. After 24 hr, the medium was removed, the cells were washed 3 times with PBS, and the intracellular ATP amount was measured using an ATP quantify kit (Perkin Elmer) and compared. The comparison results are shown in Fig. 2. When reduced coenzyme Q10 liposome or oxidized coenzyme Q10 liposome was added, the intracellular ATP increased equivalently for the both.

### (Example 2)

### <Method>

Aging promoted mouse (SAMP8, 3-week-old, male, each group N=8 - 10) models were divided into a group given a feed (CE-2, manufactured by CLEA Japan, Inc.) containing 0.5% of reduced coenzyme Q10 (containing about 1% oxidized coenzyme Q10) obtained in Production Example 1 and a group given a basic feed (CE-2, manufactured by CLEA Japan, Inc.) alone as a control group. The both groups were allowed a free access to the feed. The dose of reduced coenzyme Q10 obtained from the ingested amount of the feed and the body weight of the animal generally corresponded to 300 - 500 mg/kg/day.

The effect of reduced coenzyme Q for the learning and memory function was examined by a step-through passive avoidance learning experiment. When SAMP8 mice were placed in a light compartment of an apparatus consisting of two light and dark compartments, they enter the dark compartment since mouse prefers darkness. The mice were taught that the dark compartment was dangerous by applying an electric current (0.5 mA) for 3 sec from the floor of the dark compartment (acquisition trial). After 24 hr from the training, recall trial was performed to confirm if the mice remember the danger of entry into the dark compartment.

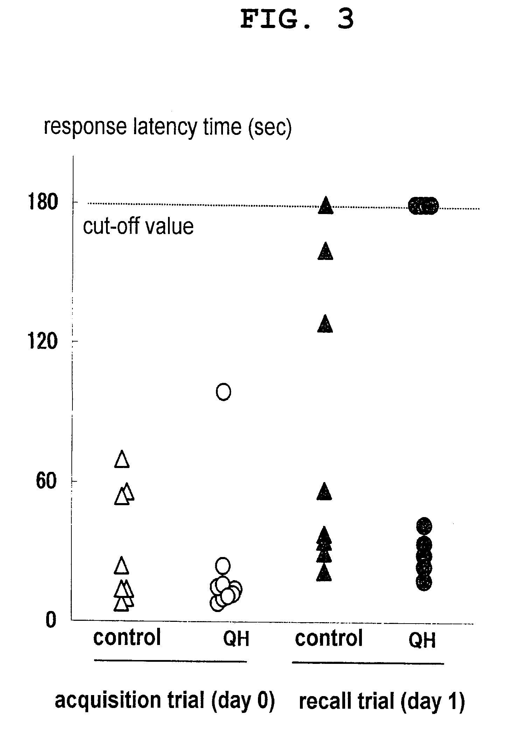

To be specific, the mice were placed in the same apparatus again, and the time of stay in the light compartment was measured, based on which the learning, memory function of the mice was compared between with and without ingestion of reduced coenzyme Q in the present invention. The mouse fed only with the control diet was used as a control group. Using the time necessary for the mice placed in the light compartment of the cage to move to the dark compartment (response latency time (sec)) as an index, the learning, memory promoting action of reduced coenzyme Q was examined. The results are shown in Fig. 3. Generally, mouse inherently moves to a dark compartment. When learning, memory is normal or when learning, memory function is promoted, the mouse can recognize or judge that the dark compartment is dangerous, and hesitates to enter the dark compartment. As a result of such normal response, the mouse stays in the light compartment, which prolongs the response latency time depending on the learning, memory ability of the mouse.

### <Results>

Fig. 3 shows the passive avoidance test results of the control group and the reduced coenzyme Q10 ingestion group after 8 weeks (11-week-old) after the start of the test. In the reduced coenzyme Q group, the number of mouse that showed a prolonged response latency time increased as compared to the control group. From the above results, a learning and memory disorder-improving effect was observed by a short time administration only of reduced coenzyme Q10.

### (Example 3) nerve cell protection effect of reduced coenzyme Q10 on hydrogen peroxide-induced cell death

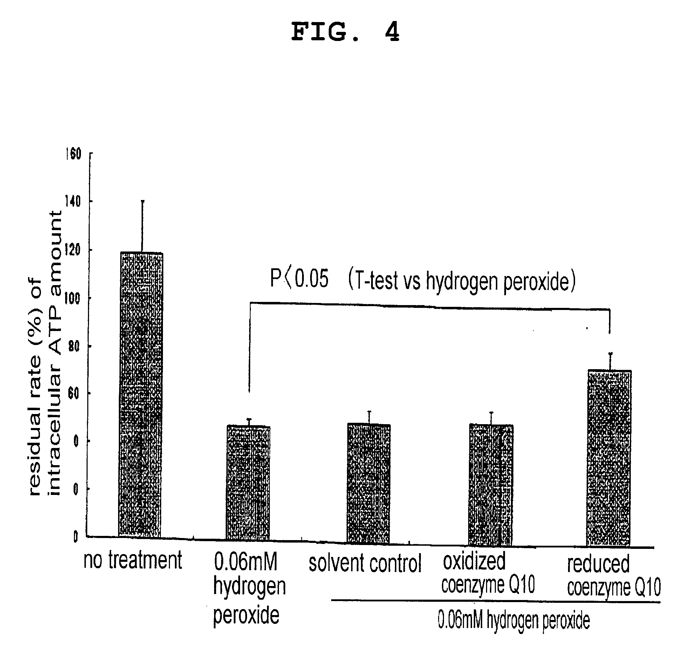

As one of the causes of the nerve cell death, active oxygen species produced for various reasons is known. Therefore, a cell death protecting action of reduced coenzyme Q10 when PC12 cells are exposed to hydrogen peroxide was examined. PC12 cells were cultured in Dulbecco's Modified Eagle Medium containing 200 units of penicillin, 0.1 µg/ml streptomycin, and 10% fetal bovine serum under 37°C, 5% CO₂ conditions. The cells were plated to a 96 well microplate at 1×104 cells/well, and cultured under the conditions of 37°C, 5% CO₂. After 24 hr, the medium was removed and substituted with Dulbecco's Modified Eagle Medium containing 200 units of penicillin, 0.1 µg/ml streptomycin and 0.5% fetal bovine serum and supplemented with reduced coenzyme Q10 and oxidized coenzyme Q10 (25, 50, 100 µg/ml). After 24 hr, the medium was removed, and the cells were exposed to hydrogen peroxide so that the final concentration in the same medium would be 0.06 mM 24 hr later, and cultured under the conditions of 37°C, 5% CO₂ for 15 min. The cells were washed 3 times with PBS, and the intracellular ATP amount was measured using an ATP quantify kit (Perkin Elmer) and compared. The comparison results are shown in Fig. 4. The cell death of PC12 cells induced by hydrogen peroxide was significantly prevented by reduced coenzyme Q10 than by oxidized coenzyme Q10.

### (Formulation Example 1) Injection

50 mg of reduced coenzyme Q10 powder, 5 g of mannitol, 100 mg of human serum albumin and 10 mg of sodium caprylate are added and the mixture is heated to about 50°C to dissolve reduced coenzyme Q in water. Then, physiological saline heated to about 50°C in advance is immediately added to about 80 mL and the mixture is sonicated to give an aqueous solution containing reduced coenzyme Q. Physiological saline is further added to measure up to the total amount of 100 mL. The solution is membrane-filtered and filled in a vial by 20 mL, which is purged with nitrogen, tightly sealed and sterilized with high-pressure vapor at 121°C for 20 min. This is freeze-dried by a conventional method to give a 10 mg/vial freeze-dried injection containing reduced coenzyme and oxidized coenzyme Q.

### (Formulation Example 2) Drink

To 5 g of reduced coenzyme Q10 powder, 1 g of glycerol, 10 g of decaglycerin fatty acid ester, 150 g of saccharide, 15 g of honey, 1 g of ascorbic acid, 0.3 g of citric acid, 2 g of aspartame and a suitable amount of flavor is added water to 1000 mL. This is sterilized at 95°C for 20 min and aseptically filled in a bottle by 100 ml to give a drink.

### (Formulation Example 3) Tablet

20 g of reduced coenzyme Q10 powder, 170 g of lactose, 8 g of cornstarch, 47 g of carboxymethylcellulose and 5 g of magnesium stearate are blended, and a tablet is produced by a conventional method.

### (Formulation Example 4) Capsule

100 g of reduced coenzyme Q10 powder, 30 g of lactose, 50 g of cornstarch, 10 g of microcrystalline cellulose, 3 g of magnesium stearate and 0.3 g of magnesium aluminometasilicate are blended, and a capsule is produced by a conventional method.

### (Formulation Example 5) Hydrophilic ointment

100 g of reduced coenzyme Q10 powder is blended with white petrolatum (1 kg) and squalene (100 g) to give hydrophilic ointment.

### Brief Description of the Drawings

Fig. 1 shows the content ratio of reduced coenzyme Q10 and oxidized coenzyme Q10 in a liposome containing reduced coenzyme Q10.
Fig. 2 shows a neural cell activating effect of reduced coenzyme Q10.
Fig. 3 is a graph showing the results of an improving effect by administration of reduced coenzyme Q on passive avoidance learning and memory disorder of SAMP8 mice, wherein Δ and ▲ show the response latency time of each mouse in the control group, and ○ and ● show the response latency time of each mouse in the reduced coenzyme Q10 administration group.
Fig. 4 shows a neural cell protecting effect of reduced coenzyme Q10.

While some of the embodiments of the present invention have been described in detail in the above, those of ordinary skill in the art can enter various modifications and changes to the particular embodiments shown without substantially departing from the novel teaching and advantages of the present invention. Such modifications and changes are encompassed in the spirit and scope of the present invention as set forth in the appended claims.

This application is based on JP 2006-091490 and JP 2006-136232 filed in Japan, the contents of which are incorporated hereinto by reference.

## Claims

1. An agent for improving a neural cell function, comprising a reduced coenzyme Q represented by the following formula (1): 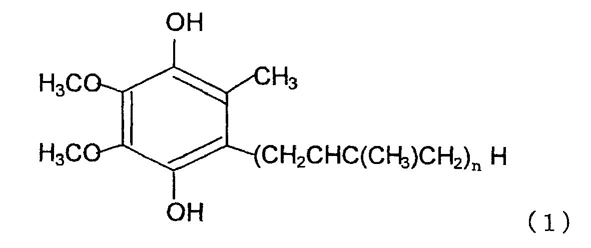 wherein n is an integer of 1 - 12, as an active ingredient.

2. The agent of claim 1, which is an agent for activating neural cells.

3. The agent of claim 2, which is used for promoting a cognitive function.

4. The agent of claim 2, which is used for improving a learning or memory disorder.

5. The agent of claim 1, which is an agent for protecting neural cells.

6. The agent of claim 2 or 5, wherein the nerve is a central nerve.

7. The agent of claim 5, which is used for the improvement or prophylaxis of a neuropathy symptom.

8. The agent of claim 7, wherein the neuropathy symptom is at least one symptom selected from the group consisting of ataxia, cognitive functional disorder, disturbance of consciousness, disturbance of sensation, hearing loss, transient blindness, homonymous hemianopsia, aphasia, alternating hemiplegia, contralateral hemiplegia, bilateral quadriplegia, dizziness, tinnitus, hearing disorder, nystagmus, double vision, tremor, numbness in a limb, and coma.

9. The agent of claim 5, which is used for the improvement or prophylaxis of a central neurological disease.

10. The agent of any one of claims 1 to 9, wherein the reduced coenzyme Q is a reduced coenzyme Q10 (wherein n is 10).

11. The agent of any one of claims 1 to 10, wherein the content of reduced coenzyme Q in the agent is 0.001 - 99 wt%.

12. The agent of any one of claims 1 to 11, further comprising an oxidized coenzyme Q represented by the following formula (2): 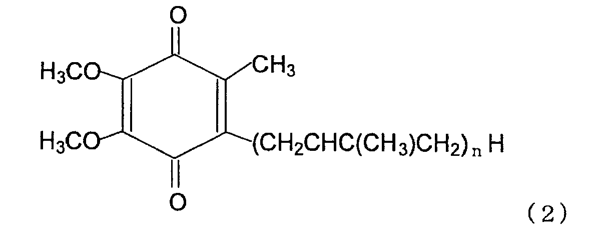 wherein n is an integer of 1 - 12.

13. The agent of claim 12, wherein the oxidized coenzyme Q is an oxidized coenzyme Q10 wherein n is 10.

14. The agent of any one of claims 1 to 13, additionally comprising at least one compound selected from the group consisting of an acetylcholinesterase inhibitor, a nerve growth factor, dopamine, edaravone, and a tissue plasminogen activator.

15. The agent of any one of claims 1 to 13, which targets a patient undergoing a treatment using at least one compound selected from the group consisting of an acetylcholinesterase inhibitor, a nerve growth factor, dopamine, edaravone and a tissue plasminogen activator, a vaccine treatment, or a gene therapy.

16. A pharmaceutical product comprising the agent of any one of claims 1 to 15 as an active ingredient.

17. A quasi-drug comprising the agent of any one of claims 1 to 15 as an active ingredient.

18. A food or functional food comprising the agent of any one of claims 1 to 15.

19. A nutrition supplement comprising the agent of any one of claims 1 to 15.

20. An animal pharmaceutical product comprising the agent of any one of claims 1 to 15 as an active ingredient.

21. A pet food, feed or prey comprising the agent of any one of claims 1 to 15.

22. A method of improving a neural cell function, comprising a step of administering an effective amount of a reduced coenzyme Q represented by the following formula (1): 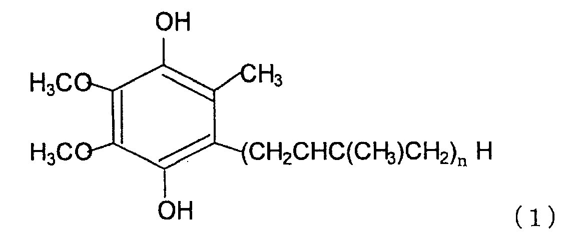 wherein n is an integer of 1 - 12, to an administration subject.

23. The method of claim 22, which is a neural cell activating method.

24. The method of claim 23, which is a method of promoting a cognitive function.

25. The method of claim 23, which is a method of improving a learning or memory disorder.

26. The method of claim 22, which is a method of protecting a neural cell.

27. The method of claim 23 or 26, wherein the nerve is the central nerve.

28. The method of claim 26, which is a method for the improvement or prophylaxis of a neuropathy symptom.

29. The method of claim 28, wherein the neuropathy symptom is at least one symptom selected from the group consisting of ataxia, cognitive functional disorder, disturbance of consciousness, disturbance of sensation, hearing loss, transient blindness, homonymous hemianopsia, aphasia, alternating hemiplegia, contralateral hemiplegia, bilateral quadriplegia, dizziness, tinnitus, hearing disorder, nystagmus, double vision, tremor, numbness in a limb, and coma.

30. The method of claim 26, which is a method for the improvement or prophylaxis of a central neurological disease.

31. The method of any one of claims 22 to 30, further comprising a step of administering an effective amount of the oxidized coenzyme Q represented by the following formula (2): 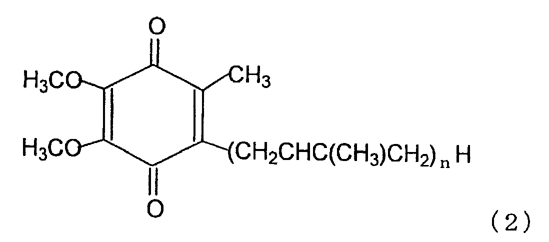 wherein n is an integer of 1 - 12, to an administration subject.

32. The method of claim 22, which is a method for increasing a coenzyme Q concentration in a nerve tissue.

33. Use of a reduced coenzyme Q represented by the following formula (1): 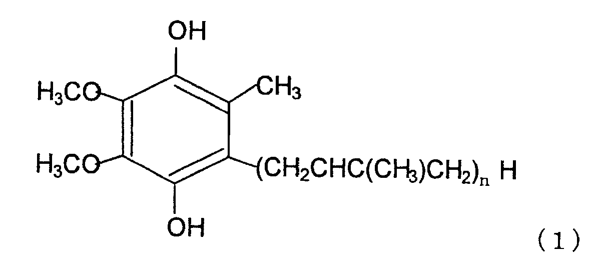 wherein n is an integer of 1 - 12, for the production of the agent of any one of claims 1 to 11.

34. Use of a reduced coenzyme Q represented by the following formula (1): 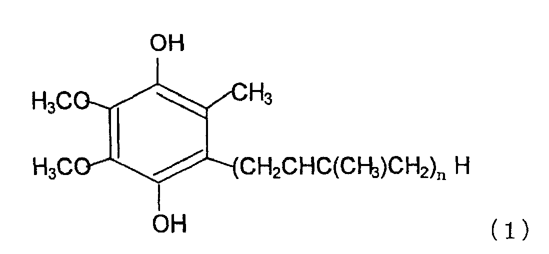 wherein n is an integer of 1 - 12, and an oxidized coenzyme Q represented by the formula (2): 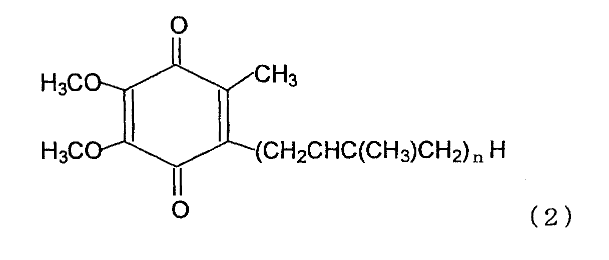 wherein n is an integer of 1 - 12, for the production of the agent of claim 12 or 13.

35. The agent of any one of claims 1 to 13, which is used for increasing the concentration of coenzyme Q in a nerve tissue.

36. A method of selling a composition for improving a neural cell function comprising a reduced coenzyme Q, which comprises use of a packaging container and/or a tool for promoting sales, both indicating that reduced coenzyme Q has a neural cell function improving effect.

37. The method of claim 36, wherein the neural cell function improving effect is a neural cell activating action.

38. The method of claim 37, wherein the neural cell activating action is a cognitive function promoting action.

39. The method of claim 37, wherein the neural cell activating action is a learning or memory disorder-improving effect.

40. The method of claim 36, wherein the neural cell function improving effect is a neural cell protecting action.

41. The method of claim 40, wherein the neural cell protecting action is a neuropathy symptom improving or preventing action.

42. The method of claim 37 or 40, wherein the nerve is a central nerve.

43. A commercial package comprising a composition comprising reduced coenzyme Q as an active ingredient and a written matter associated therewith, the written matter stating that the composition can or should be used for improving a neural cell function.
